# EUROPÄISCHE PATENTANMELDUNG

(11) **EP 4 321 108 A1**
(43) Veröffentlichungstag der Anmeldung: **14.02.2024**
(21) Anmeldenummer: 23189304.1
(22) Anmeldetag: 02.08.2023
(51) Int. Cl.: A61B 17/16

(54) **MOTORHANDSTÜCK MIT ROTIERBAREM BEDIENELEMENT SOWIE MEDIZINISCHES HANDINSTRUMENT**

(30) Priorität: 09.08.2022 DE 102022119982
(71) Anmelder: Aesculap AG, 78532 Tuttlingen (DE)
(72) Erfinder: BUERK, Andre, 78056 Villingen-Schwenningen (DE); VOGLER, Aaron, 88637 Leibertingen (DE)
(74) Vertreter: Winter, Brandl - Partnerschaft mbB

(57) **Zusammenfassung**

Die Offenbarung betrifft ein medizinisches Motorhandstück (2) zum Antreiben eines distalen Endeffektors (8), mit einem Handgriffabschnitt (4), und einem vorzugsweise hülsenförmigen Bedienelement (12), das um eine Handgrifflängsachse drehbar an einem distalen Endabschnitt des Handgriffabschnitts (4) gehalten ist und mit dem Endeffektor (8, 22) derart koppelbar ist, um eine Drehbewegung des Bedienelements (12) in eine Bewegung des Endeffektors (8, 22) zu transformieren oder eine Funktion am Endeffektor (8, 22) zu bewirken, wobei das Bedienelement (12) über zumindest ein Wälzlager (102) drehbar an dem Handgriffabschnitt (4) abgestützt ist, sowie ein medizinisches Handinstrument (1) mit einem offenbarungsgemäßen, medizinischen Motorhandstück (2) und einem Endeffektor (8, 22), welcher über einen Schaft (10, 20) mit dem Motorhandstück (2) gekoppelt ist, um Drehmoment von dem Motorhandstück (2) auf den Endeffektor (8, 22) zu übertragen.

## Beschreibung

Die Offenbarung betrifft ein medizinisches Motorhandstück zum Antreiben eines distalen Endeffektors (Werkzeug), mit einem Handgriffabschnitt, einem vorzugsweise hülsenförmigen Bedienelement, das um eine Handgrifflängsachse drehbar an einem distalen Endabschnitt des Handgriffabschnitts gehalten ist und mit dem Endeffektor derart koppelbar ist, um eine Drehbewegung des Bedienelements in eine Bewegung des Endeffektors zu transformieren oder eine Funktion am Endeffektor zu bewirken.

### Hintergrund der Offenbarung

In der modernen minimalinvasiven Chirurgie werden derartige Werkzeuge/Instrumente und dazugehörige Instrumentenhandstücke/Handinstrumente beispielsweise zur Bearbeitung von Knochen, Knorpeln bei arthroskopischen Eingriffen, in der Wirbelsäulenchirurgie und dergleichen orthopädischen/chirurgischen Behandlungen sowie zur Bearbeitung von organischem Material in der Neurochirurgie verwendet. Die Werkzeuge/Instrumente weisen ein(en) Handstück/Handgriff/Griffabschnitt und ggf. austauschbare Effektoren, wie beispielsweise Fräser, Drehmesser, einen Polierkopf oder Ähnliches auf. Der Effektor ist in einem Schaft des Werkzeugs/Instruments an dessen distalem Ende gelagert, ggf. drehbar angetrieben gelagert. Als Werkzeugantrieb ist je nach Verwendungszweck und beabsichtigter Werkzeugdrehzahl ein hydraulischer, pneumatischer, oder elektromotorischer Antrieb vorgesehen, der über einen Drehmoment-Übertragungszug innerhalb des Werkzeugs und/ oder des Handinstruments bzw. des Handgriffabschnitts mit dem Werkzeugkopf (Effektor) wirkverbunden ist. Die Antriebe können dabei im Werkzeug und/oder im Handinstrument integriert oder als externe Antriebseinheiten ausgebildet sein, die über Energieversorgungsleitungen oder Drehmoment-Übertragungsstränge mit dem Werkzeug oder dem Handinstrument gekuppelt sind.

Dabei ist es vorteilhaft, den distalen Schaftabschnitt eines Schafts von einem medizinischen Handinstrument abzuwinkeln, um so Operationen in geringem Raum durchführen zu können, beispielsweise bei Operationen an der Wirbelsäule. Anders ausgedrückt, spielt bei chirurgischen, insbesondere minimal-invasiven, Eingriffen der Bauraum der verwendeten Instrumente und eine gute Handhabbarkeit eine große Rolle. So sollen die Instrumentenschäfte insbesondere im Bereich der distalen Effektoren auf möglichst kleinem Raum aktiv (über einen Betätigungsmechanismus gewollt ausgeführt) abwinkelbar sein.

Zudem ist es von Vorteil, wenn die Werkzeuge bzw. Effektoren schnell und einfach ausgetauscht werden können, um ein schnelles Anpassen an geänderte Operationsbedingungen und/oder Arbeitsumgebungen zu gewährleisten und die Belastungen für den Patienten möglichst gering zu halten.

### Stand der Technik

Abwinkelbare Schäfte für medizinische Handinstrumente sind hinlänglich bekannt und weisen üblicherweise einen proximalen und einen abwinkelbaren distalen Schaftabschnitt auf. Der distale Schaftabschnitt und der proximale Schaftabschnitt weisen dabei beide jeweils ein schräges Ende/ eine bezüglich der jeweiligen Schaftabschnittsachse angestellte Stirnseite auf. D.h. jeweils ein Ende/ Endabschnitt/ Stirnseite des distalen und proximalen Schaftabschnitts ist nicht gerade, sondern abgeschrägt/ angestellt. Die Schrägungen/ angestellten Endabschnitte/ Stirnseiten haben jeweils den im Wesentlichen gleichen Anstellwinkel. Darum passen die Schrägungen derart zueinander, dass der proximale und der distale Schaftabschnitt in einer bestimmten Relativdrehposition einen geraden Schaft/ ein gerades Rohr bilden. Wenn nun der distale Schaftabschnitt um seine Längsachse relativ zum proximalen Schaftabschnitt rotiert und der proximale Schaftabschnitt stehen bleibt, wird der distale Schaftabschnitt durch die angestellten Stirnseiten/ Endabschnitte zwangsläufig abgewinkelt.

Ferner offenbart auch DE 10 2017 010 033 A1 eine medizinische Vorrichtung mit einer Führungseinheit, die ein Führungsrohr mit einer Längsachse, ein fest mit diesem verbundenes proximales erstes Kopplungsteil und distal einen zylindermantelförmigen Schwenkkopf aufweist, sowie mit einem im Führungsrohr axial beweglichen, mit dem Schwenkkopf verbundenen Betätigungsrohr, das durch ein proximales Bedienungselement ein Verschwenken des Schwenkkopfes bewirkt. Zur präziseren Ausrichtung eines distalen Führungselements für ein drehbares chirurgisches Werkzeug und damit der winkelmäßigen Ausrichtung des Arbeitskopfes eines solchen Werkzeugs ist das Bedienungselement um die Längsachse verschwenkbar und bewirkt unter Axialverschiebung des Betätigungsrohrs das Verschwenken des Schwenkkopfes.

Des Weiteren sind in US 7,585,300 B2 oder US 10,070,872 B2 Beispiele für chirurgische Instrumente mit einem Handstück und einem in dem Handstück aufgenommenen Schaft, an dessen distalen Ende ein Werkzeugkopf schwenkbar angelenkt ist, offenbart. Die Verschwenkung des Werkzeugkopfs kann dabei über ein an dem Handstück drehbar angeordnetes Handrad bewirkt werden. Ähnlich dazu, ist in US 8,303,594 B2 ein chirurgisches Handstück offenbart, bei welchem die Verschwenkung des Werkzeugkopfs über einen an dem Handstück angeordneten Hebel bewirkt wird.

Darüber hinaus zeigt US 9,597,093 B2 ein Werkzeug, welches mit seinem proximalen Ende drehmomentübertragend mit einer Antriebseinheit gekoppelt werden kann und an seinem distalen Ende einen Werkzeugkopf aufweist. Der Werkzeugkopf kann durch Rotation einer in dem Bereich des Werkzeugkopfs angeordneten Hülse relativ zu einem Werkzeugschaft verschwenkt werden.

Weitere Beispiele für chirurgische Handinstrumente mit rotierenden Werkzeugen, welche relativ zu einem Handstück angewinkelt / verschwenkt werden können, sind unter anderem in US 10,178,998 B2, US 10,307,180 B2 oder US 10,524,820 B2 offenbart.

Der Stand der Technik hat jedoch immer den Nachteil, dass die Werkzeuge mitsamt ihren abwinkelbaren Schäften eine aufwändige Kopplung mit der Antriebseinheit, d.h. dem Handgriffabschnitt, benötigen. Ein einfaches und schnelles Entkoppeln ist somit nicht ohne weiteres möglich.

### Zusammenfassung der Offenbarung

Es sind die Aufgaben und Ziele der Offenbarung, die Nachteile aus dem Stand der Technik zu beheben oder wenigstens zu mindern und insbesondere die Handhabbarkeit eines derartigen medizinischen bzw. chirurgischen Handinstruments, insbesondere dessen (Motor-)Handstück zu verbessern.

Die Aufgaben und Ziele werden hinsichtlich eines gattungsgemäßen Handinstruments offenbarungsgemäß durch den Gegenstand des Anspruchs 1 gelöst.

Das medizinische Handinstrument ist offenbarungsgemäß mit einem (manuell betätigbaren) Bedienelement ausgestattet, welches (um die Handstück-Längsachse) drehbar an einem stirnseitigen (distalen) Endabschnitt auf/an einem Handgriffabschnitt über zumindest ein Wälzlager / eine Wälzlagerstelle abgestützt ist.

Somit kann eine (Axial-)Lagerung des Bedienelements mit sehr geringem Spiel verwirklicht werden. Ferner können optimale Laufeigenschaften erzielt werden, da kein Haftgleiteffekt auftritt.

Vorteilhafte Ausführungsformen sind in den Unteransprüchen beansprucht und werden nachfolgend erläutert.

Vorzugsweise kann das Motorhandstück über einen Schaft mit dem Endeffektor koppelbar sein und das Bedienelement kann bei dessen Drehbewegung in einer ersten Drehrichtung die Bewegung des Endeffektors, in Form eines Abwinkelns des Endeffektors relativ zu dem Schaft, bewirken. Dabei kann es ferner von Vorteil sein, wenn der Endeffektor eine erste Kupplungsvorrichtung aufweist und der Schaft eine zweite Kupplungsvorrichtung aufweist, welche in einem Kupplungszustand miteinander in Wirkeingriff stehen, wobei eine Drehbewegung des Bedienelements in einer der ersten Drehrichtung entgegengesetzten, zweiten Drehrichtung den Wirkeingriff zwischen der ersten Kupplungsvorrichtung und der zweiten Kupplungsvorrichtung löst.

Anders ausgedrückt kann das Bedienelement relativ zu dem (fest in einer Hand aufgenommen) Handgriffabschnitt in einer ersten Drehrichtung und in einer der ersten Drehrichtung entgegengesetzten, zweiten Drehrichtung drehbar sein, um bei einer Drehung des Bedienelements in der ersten Drehrichtung ein Abwinkeln des Effektorabschnitts relativ zu dem Schaft (und bei Weiterdrehen in der ersten Drehrichtung wieder ein Geradeausrichten des Schafts) und bei einer Drehung des Bedienelements in der zweiten Drehrichtung ein Lösen der Kopplung zwischen dem Effektorabschnitt und dem Schaft (ohne Abwinkelbewegungen des distalen Schaftabschnitts) zu bewirken.

Dabei kann es zweckmäßig sein, dass der Handgriffabschnitt, insbesondere das Bedienelement, zumindest eine Markierung /Indikator, vorzugsweise in Form einer Beschriftung und/oder eines Piktogramms, zur Anzeige der Funktion der ersten und/oder der zweiten Drehrichtung aufweist.

Ferner kann an dem Handgriffabschnitt, insbesondere an dem Bedienelement, ein Verriegelungselement, vorzugsweise in Form eines in Richtung distal verlagerbaren Verriegelungsschiebers, angeordnet sein, um in einer Verriegelungsposition die Drehung des Bedienelements relativ zu dem Handgriffabschnitt zu verriegeln. Anders ausgedrückt kann der Verriegelungsschieber vorgesehen sein, um das Abwinkeln des Effektorabschnitts relativ zu dem Schaft bei einem bestimmten/gewählten Winkel zu verriegeln / festzustellen.

Bei einer besonders vorteilhaften Ausgestaltung kann das Bedienelement drehbar auf einem Trägerelement des Handgriffabschnitts abgestützt sein. Das Trägerelement kann dabei vorzugsweise in Form einer Hohlwelle bzw. einer Hülse ausgebildet sein.

Gemäß einer bevorzugten Weiterbildung kann ein Außenring des Wälzlagers einstückig mit dem Bedienelement ausgebildet sein. Anders ausgedrückt, kann eine Laufbahn des Außenrings des Wälzlagers in das Bedienelement integriert sein. Hierfür kann vorzugsweise auf einer Innenumfangsfläche des Bedienelements eine umlaufende Nut ausgebildet sein, in welcher Wälzkörper des Wälzlagers aufgenommen sind. Dadurch kann der Lageraufbau besonders kompakt gestaltet werden.

Ferner kann es von Vorteil sein, wenn ein Innenring des Wälzlagers einstückig mit dem Trägerelement ausgebildet ist. Mit anderen Worten, kann eine Laufbahn des Innenrings des Wälzlagers in das Bedienelement integriert sein. Hierfür kann vorzugsweise auf einer Außenumfangsfläche des Bedienelements eine umlaufende Nut ausgebildet sein, in welcher Wälzkörper des Wälzlagers aufgenommen sind, was die Kompaktheit des Lageraufbaus weiter erhöht.

In einer vorteilhaften Ausführungsform kann das Bedienelement eine Einfüllöffnung zur Montage von Wälzkörpern des Wälzlagers aufweisen.

Offenbarungsgemäß kann das zumindest eine Wälzlager als Kugel-, Nadel-, oder Zylinderrollenlager ausgeführt sein.

Gemäß einer bevorzugten Weiterbildung kann das Bedienelement über zwei Wälzlager an dem Handgriffabschnitt abgestützt sein.

Ferner betrifft die Offenbarung ein medizinisches Handinstrument mit einem offenbarungsgemäßen, medizinischen Motorhandstück und einem Endeffektor, welcher über einen Schaft mit dem Motorhandstück gekoppelt ist, um Drehmoment von dem Motorhandstück auf den Endeffektor zu übertragen.

Mit anderen Worten betrifft die Offenbarung ein kugelgelagertes Bedienelement eines medizinischen Handinstruments. Das rotierbare Bedienelement des Handstücks weist mindestens eine vollkugelige Lagerstelle auf. Dabei handelt es sich um einen besonders kompakten Lageraufbau, da die Laufbahn des Innenrings in das Trägerelement (Träger) des Handgriffabschnitts integriert ist und die Laufbahn des Außenrings in das Bedienelement (Drehhülse) integriert ist. Das offenbarungsgemäße Handinstrument ermöglicht eine Axiallagerung des Bedienelements mit sehr geringem Spiel. Zudem kann das Auftreten des Haftgleiteffekts (Stick-Slip-Effekt) unterbunden werden. D.h. es können optimale Laufeigenschaften gewährleistet werden.

### Kurzbeschreibung der Figuren

Die Offenbarung wird nachfolgend anhand bevorzugter Ausführungsbeispiele mit Hilfe von Figuren näher erläutert. Es zeigen:
- Fig. 1: eine perspektivische Ansicht eines medizinischen Handinstruments gemäß einer bevorzugten Ausführungsform der vorliegenden Offenbarung;
- Fig. 2: eine weitere perspektivische Ansicht des medizinischen Handinstruments gemäß der bevorzugten Ausführungsform der vorliegenden Offenbarung;
- Fig. 3: eine Teillängsschnittansicht eines Motorhandstücks des medizinischen Handinstruments gemäß der bevorzugten Ausführungsform;
- Fig. 4: eine Querschnittansicht des Motorhandstücks des medizinischen Handinstruments gemäß der bevorzugten Ausführungsform;
- Fig. 5: eine Teillängsschnittansicht eines distalen Endabschnitts des medizinischen Handinstruments gemäß der bevorzugten Ausführungsform in einer geraden Schaftform;
- Fig. 6: eine Teillängsschnittansicht des distalen Endabschnitts des medizinischen Handinstruments gemäß der bevorzugten Ausführungsform in einer abgewinkelten Schaftform;
- Fig. 7: eine Teillängsschnittansicht des distalen Endabschnitts des medizinischen Handinstruments gemäß der bevorzugten Ausführungsform in einem Freigabezustand;
- Fig. 8: eine isometrische Perspektivansicht des Motorhandstücks des medizinischen Handinstruments gemäß der bevorzugten Ausführungsform;
- Fig. 9: eine Teillängsschnittansicht des Motorhandstücks des medizinischen Handinstruments gemäß der bevorzugten Ausführungsform;
- Fig. 10: eine perspektivische Längsschnittansicht des medizinischen Handinstruments gemäß einer Modifikation der bevorzugten Ausführungsform;
- Fig. 11: eine Detailansicht einer Abgleitkulisse des medizinischen Handinstruments gemäß der Modifikation der bevorzugten Ausführungsform;
- Fig. 12: eine Teillängsschnittansicht des Handgriffabschnitts des medizinischen Handinstruments gemäß der bevorzugten Ausführungsform; und
- Fign. 13 und 14: Schnittansichten des Handinstruments gemäß der bevorzugten Ausführungsform.

Die Figuren sind schematischer Natur und dienen lediglich dem Verständnis der Offenbarung. Gleiche Elemente sind mit denselben Bezugszeichen versehen.

### Detaillierte Beschreibung einer bevorzugten Ausführungsform

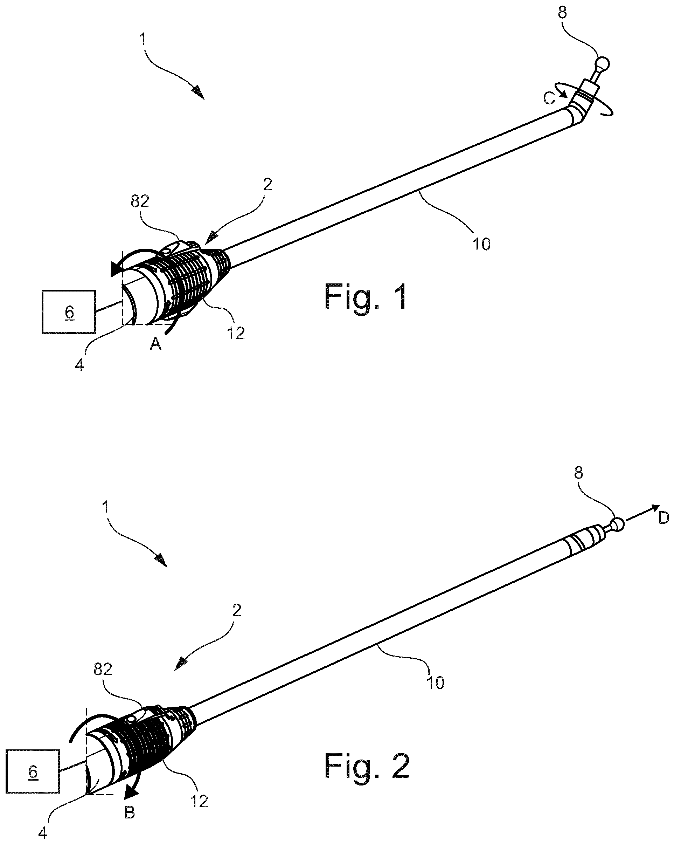

Figur 1 zeigt perspektivisch ein medizinisches Handinstrument 1 gemäß einer bevorzugten Ausführungsform der vorliegenden Offenbarung. Das medizinische Handinstrument 1 weist dabei ein medizinisches Motorhandstück 2 auf, welches einen proximalen Handgriffabschnitt 4 aufweist und welches, wie in Fig. 1 schematisch dargestellt, mit einer Antriebseinheit 6 gekoppelt werden kann. Alternativ kann die Antriebseinheit 6 auch in dem Motorhandstück 2 aufgenommen sein und über einen Energieversorgungsanschluss mit Energie versorgt werden.

Ferner weist das Handinstrument 1 ein distales Werkzeug (Endeffektor/Effektorabschnitt) 8 auf, welches über einen (Werkzeug-)Schaft 10 mit dem Motorhandstück 2, insbesondere mit dem Handgriffabschnitt 4, gekoppelt werden oder von diesem entkoppelt werden kann. Das Werkzeug/Endeffektor 8 kann dabei beispielsweise als Fräser, Bohrer oder Polierkopf ausgeführt sein.

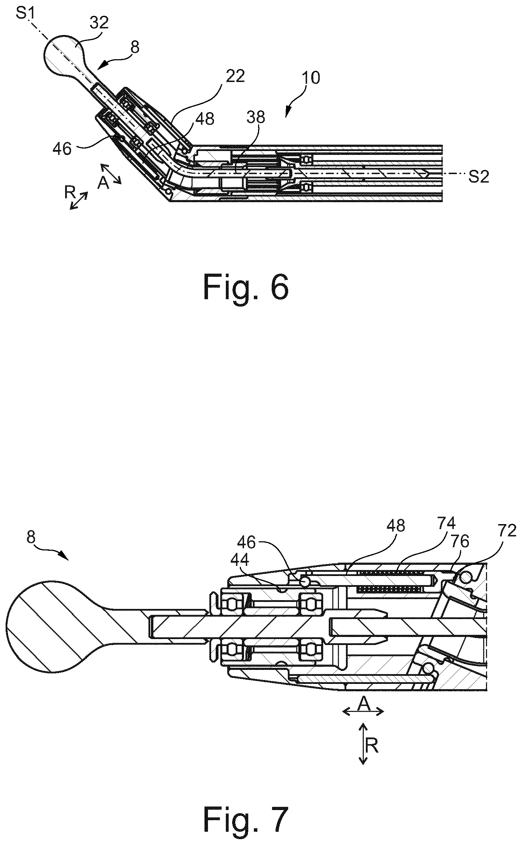

Wenn der Schaft 10 mit dem Handgriffabschnitt 4 gekoppelt ist, wird ein Drehmoment von der Antriebseinheit 6 über einen in dem Handinstrument 1, insbesondere dem Handgriffabschnitt 4 und dem Schaft 10, angeordneten Drehmoment-Übertragungszug hin zu dem Werkzeug 8 übertragen, um dieses in Drehung zu versetzen. Bei Verwendung des Handinstruments 1 im Rahmen eines chirurgischen Eingriffs (Operation) bzw. einer medizinischen Behandlung ist es bekannt, dass das Werkzeug 8 relativ zu dem Schaft 10 abgewinkelt werden kann (in Fig. 1 durch den Pfeil C angedeutet). D.h. das Werkzeug 8 und der Schaft 10 können, wie nachstehend näher beschrieben, so zueinander abgewinkelt werden, dass eine Längsachse S1 des Werkzeugs 8 und eine Schaftlängsachse S2 des Schafts 10 miteinander einen Winkel ungleich 180° (gerade Schaftausrichtung), insbesondere kleiner als 180°, ausbilden (vgl. Fig. 6).

Wie in Fig. 1 gezeigt, ist hierfür bei dem offenbarungsgemäßen Handinstrument 1 ein hülsenförmiges Bedienelement 12 vorgesehen. Das Bedienelement 12 ist dabei an einem distalen Endabschnitt des Handgriffabschnitts 4 angeordnet und um eine Handgrifflängsachse in einer ersten Drehrichtung A und in einer der ersten Drehrichtung A entgegengesetzten, zweiten Drehrichtung B drehbar. Mit anderen Worten ausgedrückt, weist das Motorhandstück 2 den Handgriffabschnitt 4 und das distal angeordnete Bedienelement 12 auf, welche koaxial zueinander angeordnet sind, wobei das Bedienelement 12 relativ zu dem Handgriffabschnitt 4 gedreht werden kann.

Eine Drehung des Bedienelements 12 aus einer neutralen Nullstellung, bei welcher das Werkzeug 8 nicht relativ zu dem Schaft 10 abgewinkelt ist, in der ersten Drehrichtung A bewirkt dabei, wie in Fig. 1 gezeigt, ein Abwinkeln des Werkzeugs 8 relativ zu dem Schaft 10 (beispielsweise infolge der zur Schaftlängsachse schräg angestellten Stirnflächen der beiden Schaftabschnitte, wie dies im genannten Stand der Technik bereits vorgesehen ist). Die erste Drehrichtung A ist bei dem Handinstrument 1 gemäß der vorliegenden Offenbarung als eine Drehung des Bedienelements 12 relativ zu dem Handgriffabschnitt 4 nach links definiert. Anders ausgedrückt entspricht die erste Drehrichtung A in einer distalen Draufsicht auf das Werkzeug 8 einer Drehung des Bedienelements 12 im Uhrzeigersinn.

Wie in Fig. 2 gezeigt, kann das Bedienelement 12 bei dem offenbarungsgemäßen Handinstrument 1 auch in der zweiten Drehrichtung B, welche der ersten Drehrichtung A entgegengesetzt ist, relativ zu dem Handgriffabschnitt 4 gedreht werden. D.h. die zweite Drehrichtung B ist in der Draufsicht auf das Werkzeug 8 als eine Drehung des Bedienelements 12 entgegen dem Uhrzeigersinn bzw. als eine Drehung nach rechts definiert. Wie durch den Pfeil D in Fig. 2 angedeutet, bewirkt eine Drehung des Bedienelements 12 in der zweiten Drehrichtung B ein Lösen der Kopplung des Werkzeugs 8 mit dem Schaft 10. Somit kann, wie nachstehend näher erläutert, durch eine Drehung des Bedienelements 12 in der zweiten Drehrichtung C ein einfacher und schneller Werkzeugwechsel gewährleistet werden.

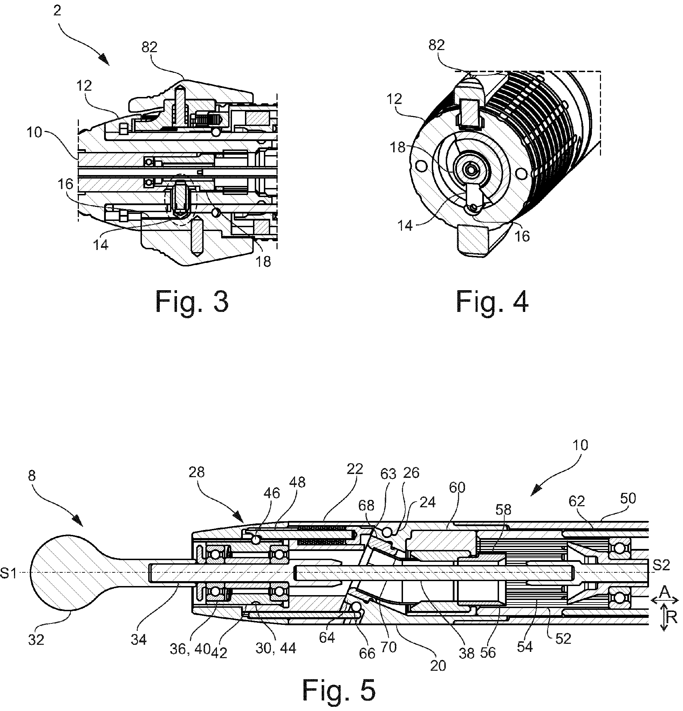

Fign. 3 und 4 zeigen Teilschnittansichten des Handinstruments 1 gemäß der bevorzugten Ausführungsform. Zur Übertragung der Drehbewegung des Bedienelements 12 auf den Schaft 10 ist in radialer Richtung des Bedienelements 12 ein Verstellstift 14 angeordnet. Ein radial außenliegender Endabschnitt des Verstellstifts 14 ist dabei in einer Axialnut 16, welche auf einer Innenumfangsfläche des Bedienelements 12 ausgebildet ist, aufgenommen. Auf einer radialen Innenseite ist der Verstellstift 14 in einer Drehübertragungshülse 18 aufgenommen. Die Drehübertragungshülse 18 ist dabei drehfest mit dem Schaft 10 verbunden, so dass eine Bewegung des Verstellstifts 14 in Umfangsrichtung eine Drehung der Drehübertragungshülse 18 und des Schafts 8 bewirkt. Anders ausgedrückt, bewegt sich bei Verdrehung des Bedienelements 12 in der ersten Drehrichtung A oder in der zweiten Drehrichtung B der Verstellstift 14 in der Axialnut 16 mit dem Bedienelement 10 mit und bewirkt je nach Drehrichtung das Abwinkeln bzw. den Auswurf des Werkzeugs 8.

Fig. 5 zeigt einen Längsquerschnitt eines distalen Endabschnitts des Handinstruments 1 in einer geraden Schaftform. D.h. das Bedienelement 12 ist nicht relativ zu dem Handgriffabschnitt 4 verdreht (Nullstellung). Demzufolge sind das Werkzeug 8 und der Schaft 10 nicht abgewinkelt und die Längsachse S1 des Werkzeugs 8 ist kollinear zu der Schaftlängsachse S2. Wie in Fig. 5 zu erkennen, weist der Schaft 10 einen, dem Handgriffabschnitt 4 zugewandten und mit diesem koppelbaren, proximalen Schaftabschnitt 20 und einen mit dem Werkzeug 8 koppelbaren distalen Schaftabschnitt 22 auf. Wenn das Bedienelement 12 nicht relativ zu dem Handgriffabschnitt 4 verdreht ist und der Schaft 10 in der geraden Schaftform vorliegt, sind der proximale Schaftabschnitt 20 und der distale Schaftabschnitt 22 demzufolge in einer Linie angeordnet bzw. weisen einen Winkel von 0° zueinander auf. Der proximale Schaftabschnitt 20 ist dabei im Wesentlichen rohrförmig ausgestaltet und weist an seinem distalen Endabschnitt eine in Schaftlängsachse S2 angestellte Stirnseite 24 auf. Der distale Schaftabschnitt 22 ist ebenfalls annähernd rohrförmig ausgeführt. Das Rohr läuft zum distalen Ende hin spitzförmig zu. Der distale Schaftabschnitt 22 weist an seinem proximalen Endabschnitt eine in Schaftlängsachse S2 angestellte Stirnseite 26 auf.

Die angestellten Stirnseiten 24, 26 weisen jeweils einen Anstellwinkel von vorzugsweise 22,5° zu einer Normalenebene zur Schaftlängsachse S2 auf. Wenn der Schaft 10 in einer geraden Schaftform bzw. ausgestreckt ist, sind die beiden angestellten Stirnseiten 24, 26 derart zueinander versetzt, dass sich die langen Enden der angestellten Stirnseiten 24, 26 in Relation zur Schaftlängsachse S2 gegenüberliegen. Die angestellten Stirnseiten 24, 26 liegen aufeinander auf. Die angestellten Stirnseiten 24, 26 müssen dabei nicht zwangsläufig einen Anstellwinkel von 22,5° aufweisen. Es sind auch Anstellwinkel von beispielsweise 10°, 18°, 30°, 45° oder jeder andere Anstellwinkel denkbar.

Wie vorstehend erwähnt, ist der distale Schaftabschnitt 22 ausgebildet, mit dem Werkzeug 8 gekoppelt zu werden. D.h. der distale Schaftabschnitt 22 stellt eine Werkzeugaufnahme dar, wobei das Werkzeug 8 in der Werkzeugaufnahme aufgenommen ist und relativ zu der Werkzeugaufnahme, d.h. dem distalen Schaftabschnitt 22, drehbar gelagert ist.

Hierfür ist in dem distalen Schaftabschnitt 22 eine erste Kupplungsvorrichtung 28 angeordnet. An dem Werkzeug 8 ist eine zweite Kupplungsvorrichtung 30 vorgesehen, die in einem Kupplungszustand durch Zusammenwirken mit der ersten Kupplungsvorrichtung 28 eine Kupplung zwischen dem Werkzeug 8 und dem distalen Schaftabschnitt 22 realisiert, um das Werkzeug 8 im distalen Schaftabschnitt 22 in Axialrichtung A zu fixieren.

Wie vorstehend erwähnt, schließen die Längsachse S1 des Werkzeugs 8 bzw. des distalen Schaftabschnitts 22 und die Schaftlängsachse S2, d.h. die Längsachse des proximalen Schaftabschnitts 20 in Fig. 5 einen Winkel von 0° ein. Zudem ist in Fig. 5 ein Kupplungszustand zwischen dem Werkzeug 8 und dem distalen Schaftabschnitt 22 gezeigt. Das heißt, dass die erste Kupplungsvorrichtung 28, die im/am distalen Schaftabschnitt 22 angebracht ist, und die zweite Kupplungsvorrichtung 30, die am Werkzeug 8 vorgesehen ist, miteinander in Eingriff stehen bzw. zusammenwirken. Das Werkzeug 8 weist einen Werkzeugkopf/Effektor 32, beispielsweise einen Fräskopf, und einen Werkzeugschaft 34 auf. Der Werkzeugkopf 32 und der Werkzeugschaft 34 sind drehfest miteinander verbunden.

In Fig. 5 ist weiterhin zu erkennen, dass der Werkzeugschaft 34 mithilfe einer Wälzlagereinheit 36 in dem distalen Schaftabschnitt 22 drehbar gelagert ist. Durch den proximalen Schaftabschnitt 20 erstreckt sich eine Antriebswelle 38, welche drehfest mit dem Werkzeugschaft 34 verbunden ist und in Form eines Drehmomentübertragungszugs Drehmoment von der Antriebseinheit 6 auf den Werkzeugschaft 34 aufbringt. Der Werkzeugschaft 34 dreht sich infolge dieser Drehmomentbeaufschlagung relativ zu dem distalen Schaftabschnitt 22. Die Wälzlagereinheit 36 weist zumindest ein, hier genau zwei, voneinander in Axialrichtung A beabstandete Wälzlager 40, genauer gesagt Kugellager, auf, die alternativ aber auch als Gleitlager ausgebildet sein können. Die Wälzlager 40 sind in einem Lagergehäuse 42, das Teil der Wälzlagereinheit 36 ist, aufgenommen.

Die zweite Kupplungsvorrichtung 30 ist in das Lagergehäuse 42 eingebracht und damit nicht direkt an dem Werkzeug 8 vorgesehen. Die Wälzlagereinheit 36 und damit auch das Lagergehäuse 42 ist an dem Werkzeugschaft 34 in Axialrichtung A fixiert angeordnet. Alternativ dazu wäre es aber auch denkbar, dass die zweite Kupplungsvorrichtung 30 direkt an dem Werkzeugschaft 34 vorgesehen ist. Die zweite Kupplungsvorrichtung 30 ist als eine sich in Umfangsrichtung des Lagergehäuses 42 durchgängig ersteckende bzw. umlaufende Axialsicherungsnut 44 ausgebildet. Die Axialsicherungsnut 44 hat vorzugsweise einen halbkreisförmigen oder kreissegmentförmigen Querschnitt.

Die erste Kupplungsvorrichtung 28, die in dem distalen Schaftabschnitt 22 vorgesehen ist, weist zumindest eine Verriegelungskugel 46 auf. Der Durchmesser der Verriegelungskugel 46 ist so gewählt, dass die Verriegelungskugel 46 in der Axialsicherungsnut 44 aufnehmbar ist. Vorzugsweise umgibt die Axialsicherungsnut 44 zumindest einen die Axialsicherungsnut 44 kontaktierenden Abschnitt der Verriegelungskugel 46 vollumfänglich. Die Verriegelungskugel 46 wird im Kupplungszustand an ihrer der Axialsicherungsnut 44 gegenüberliegenden Seite von einem distalen Ende eines Raststiftes/Schiebers 48 in der Axialsicherungsnut 44 gehalten. Der Raststift 48 ist ein Teil der ersten Kupplungsvorrichtung 28. Der Raststift 48 ist in Radialrichtung R fixiert. Der Raststift 48 ist in Axialrichtung verschieblich bzw. beweglich in dem distalen Schaftabschnitt 22 angeordnet.

In dem in Fig. 5 gezeigten Kupplungszustand ist also die Verriegelungskugel 46 in der Axialsicherungsnut 44 aufgenommen und wird von dem Raststift 48 in Radialrichtung in der Axialsicherungsnut 44 gehalten. Diese Position des Raststiftes 48 in Axialrichtung A wird als Kupplungsposition bezeichnet. Eine Kante 63 am distalen Ende des proximalen Schaftabschnittes 20 verhindert eine Bewegung des Raststiftes 48 in Axialrichtung hin zu dem proximalen Schaftabschnitt 20.

Der proximale Schaftabschnitt 20 weist ein feststehendes Außenrohr 50, ein Hohlrad 52 mit einer Innenverzahnung 54, ein Ritzel 56 mit einer Außenverzahnung 58 und eine exzentrische Sicherungsbuchse 60 auf. Das Hohlrad 52 ist innerhalb des Außenrohrs 50 positioniert und die Längsachse des Außenrohrs 50 entspricht der Längsachse des Hohlrads 52. Das Außenrohr 50 und das Hohlrad 52 sind also konzentrisch angeordnet. Das Hohlrad 52 ist über eine in dem proximalen Schaftabschnitt 20 aufgenommene Hohlwelle 62 mit der Drehübertragungshülse 18, d.h. mit dem Bedienelement 12, verbunden.

Wie vorstehend erwähnt, ist das Außenrohr 50 als feststehendes Rohr ausgeführt und bewegt sich demnach nicht. Das distale Ende des Außenrohrs 50 weist die angestellte Stirnseite 24 auf. Das distale Ende des Außenrohrs 50 weist weiterhin eine Aufnahmebohrung 64 und einen Aufnahmezapfen für ein Wälzlager 66 auf. Das Außenrohr 50 weist eine Rille/ Nut für die Kugeln des Wälzlagers 66 auf. Auf dem Wälzlager 66 ist der distale Schaftabschnitt 22 gelagert.

Die Innenverzahnung 54 kämmt mit der Außenverzahnung 58 des Ritzels 56. Dadurch wird eine Rotation des Hohlrads 52, die durch das Bedienelement 12 gesteuert wird, auf das Ritzel 56 übertragen. Die Drehrichtung des Ritzels 56 ist dabei gleichgesetzt der Drehrichtung des Hohlrads 52. Das Ritzel 56 wird vom Hohlrad 52 angetrieben, das Ritzel 56 dreht sich jedoch in der exzentrischen Sicherungsbuchse 60. Die Sicherungsbuchse 60 ist exzentrisch zum Hohlrad 52 angeordnet. D.h. die Längsachse der exzentrischen Sicherungsbuchse 60 ist zwar parallel zur Längsachse des Hohlrads 52, die Längsachsen liegen aber nicht übereinander bzw. versetzt zueinander. Der distale Schaftabschnitt 22 weist eine Verstellbuchse 68 auf. Die Verstellbuchse 68 ist in der Aufnahmebohrung 64 des proximalen Schaftabschnitts 20 gelagert. Die Verstellbuchse 68 ist über einen flexiblen Silikonschlauch 70 mit dem Ritzel 56 derart verbunden, dass eine Rotation des Ritzels 56 auf die Verstellbuchse 68 übertragen wird. Hierfür ist der flexible Silikonschlauch 70 an der Verstellbuchse 68 und dem Ritzel 56 beispielsweise durch Schweißen oder Kleben befestigt. Die Verstellbuchse 68 ist ferner über einen Mitnahmezapfen (nicht dargestellt) mit dem distalen Schaftabschnitt 22 formschlüssig verbunden ist, so dass eine Rotation der Verstellbuchse 68 auf den distalen Schaftabschnitt 22 übertragen wird.

Fig. 6 zeigt einen Längsquerschnitt durch den distalen Endabschnitt des Schafts 10, wobei der distale Schaftabschnitt 22 zu dem proximalen Schaftabschnitt 20 bei einem jeweiligen Anstellwinkel der beiden Stirnseiten 24, 26 um 22,5° folglich um 45° abgewinkelt ist. Der distale Schaftabschnitt 22 ist im Vergleich zur Stellung in Fig. 5 um 180° um die eigene Längsachse S1 gedreht. Die angestellten Stirnseiten 24, 26 liegen in dieser Stellung wieder komplett / flächig aufeinander auf. Durch die Drehung des distalen Schaftabschnitts 22 sind die langen Enden der angestellten Stirnseiten 24, 26 aber nebeneinander positioniert. Die Anstellwinkel der angestellten Stirnseiten 24, 26 addieren sich somit. Dadurch wird der distale Schaftabschnitt 22 im Vergleich zum proximalen Schaftabschnitt 20 um den doppelten Anstellwinkel der angestellten Stirnseiten 24, 26 abgewinkelt.

In der in Fig. 6 gezeigten Stellung, ist der Mitnahmezapfen gegenüber der Sicherungsbuchse 60 angeordnet. D.h. die Verstellbuchse 68 hat sich von der ausgestreckten Position zur maximalen Abwinklung um 180° gedreht. Die 45°-Position stellt für diese Konstruktion den Umkehrpunkt dar. Die Verstellbuchse 68 hat sich in dieser Position um 180° gedreht. Bei weiterer Drehung des Hohlrads 52, d.h. des Bedienelements 12, könnte sich der distale Schaftabschnitt 22 in einer alternativen Ausführungsform wieder in die Ausgangsstellung (Nullstellung) zurück drehen. In der vorliegenden, bevorzugten Ausführungsform erfolgt allerdings eine Rückstellung zur Nullstellung lediglich durch Drehrichtungsumkehr des Bedienelements 12. Damit sozusagen ein Überdrehen des Bedienelements 12 um über 180° unterbunden bzw. blockiert wird, sind entsprechende Anschläge im Handstück vorgesehen.

Ferner ist in Fig. 6 zu erkennen, dass die Verriegelungskugel 46 auch dann, wenn der distale Schaftabschnitt 22 in der maximal einstellbaren Winkelstellung bzw. maximale Abwinklung bezüglich des proximalen Schaftabschnitts 20 abgewinkelt ist, durch den Raststift 48 in Radialrichtung R in der Axialsicherungsnut 44 gehalten ist. Auf diese Weise ist das Werkzeug 8 in dem distalen Schaftabschnitt durch den Eingriff zwischen erster Kupplungsvorrichtung 28 und zweiter Kupplungsvorrichtung 30 auch in dieser Winkelstellung in Axialrichtung A gesichert.

Der Drehmomentstrang, insbesondere die Antriebswelle 38, hin zum Werkzeugschaft 34, der im Übergangsbereich zwischen dem distalen Schaftabschnitt 22 und dem proximalen Schaftabschnitt 20 angeordnet ist, ist flexibel. Dieser flexible Abschnitt des Drehmomentstrangs erlaubt, dass der distale Abschnitt des Werkzeugschaftes 34 mit Werkzeugkopf 32 zusammen mit dem distalen Schaftabschnitt 22 relativ zum Drehmomentstrang im proximalen Schaftabschnitt 20 abgewinkelt werden kann. Gleichzeitig ist der flexible Abschnitt des Drehmomentstrangs so ausgeführt, dass er ein Drehmoment, das auf einen proximalen Abschnitt des Werkzeugschaftes 34 ausgeübt wird, weiterhin auf den Werkzeugkopf 32 übertragen kann.

Fig. 7 ist eine Längsschnittansicht des distalen Endabschnitts des Schafts 10 in einem Freigabezustand zwischen dem Werkzeug 8 und dem distalen Schaftabschnitt 22. D.h. in der in Fig. 7 gezeigten Stellung stehen die die erste Kupplungsvorrichtung 28 und die zweite Kupplungsvorrichtung 30 nicht in Wirkeingriff miteinander, so dass das Werkzeug 8 entnommen bzw. gewechselt werden kann. Um den Wirkeingriff der ersten Kupplungsvorrichtung 28 und der zweiten Kupplungsvorrichtung 30 zu lösen, wird das Bedienelement 12 in der zweiten Drehrichtung B gedreht (vgl. Fig. 2). Wie nachstehend näher erläutert, ist die Verriegelungskugel 46 dabei nicht mehr in der Axialsicherungsnut 44 aufgenommen. Stattdessen ist sie von dem Raststift 48 in Radialrichtung R gegen eine Außenumfangsfläche des Lagergehäuses 42 gehalten.

Damit sich die Verriegelungskugel 46 ausgehend von dem, in Fig. 5 gezeigten Kupplungszustand, aus der Axialsicherungsnut 44 heraus in den Freigabezustand bewegen kann, muss der Raststift 48 sich in Axialrichtung A hin zu dem proximalen Schaftabschnitt 20 bewegen. Dies wird im Kupplungszustand durch die umlaufende Kante 63 des proximalen Schaftabschnitts 20 verhindert. Die Kante 63 ist jedoch an einer Stelle einer Raststift-Aufnahmevertiefung 72 unterbrochen. Wenn das Bedienelement 12 in der zweiten Drehrichtung B gedreht wird, rotiert der distale Schaftabschnitt 22 relativ zu dem proximalen Schaftabschnitt 20 in einer dem Abwinkeln entgegengesetzten Richtung, beispielsweise um (-)18°, bis der Raststift 48 relativ zu dem proximalen Schaftabschnitt 20 so positioniert ist, dass er in Umfangsrichtung auf einer Höhe mit der Raststift-Aufnahmevertiefung 72 ist. Ein Vorspannelement 74, das ein Teil der ersten Kupplungsvorrichtung 28 ist, drückt den Raststift 48 in Axialrichtung A zum proximalen Schaftabschnitt 20 hin. Somit schiebt das Vorspannelement 74 den Raststift 48, genauer gesagt dessen proximales Ende, das als Rastvorsprung 76 ausgebildet ist, in die Raststift-Aufnahmevertiefung 72 hinein (gezeigt in Fig. 7). Die Position, in der der Raststift 48 ist, wenn sein Rastvorsprung 76 in die Raststift-Aufnahmevertiefung 72 eingreift, wird als der Freigabezustand bzw. Freigabeposition bezeichnet.

In der Freigabeposition liegt das distale Ende des Raststifts 48 nicht mehr der Axialsicherungsnut 44 gegenüber. Damit wird die Verriegelungskugel 46 in Radialrichtung R nicht in der Axialsicherungsnut 44 gehalten. Das Werkzeug 8 ist somit relativ zum distalen Schaftabschnitt 22 nicht mehr in Axialrichtung A fixiert. Wird nun, ausgehend vom Kupplungszustand, eine Zugkraft (in Axialrichtung A) auf das distale Ende des Werkzeugs 8 aufgebracht, löst sich die Verriegelungskugel 46 aus der Axialsicherungsnut 44. Auf diese Weise kann das Werkzeug 8 von dem distalen Schaftabschnitt 22 entkuppelt werden.

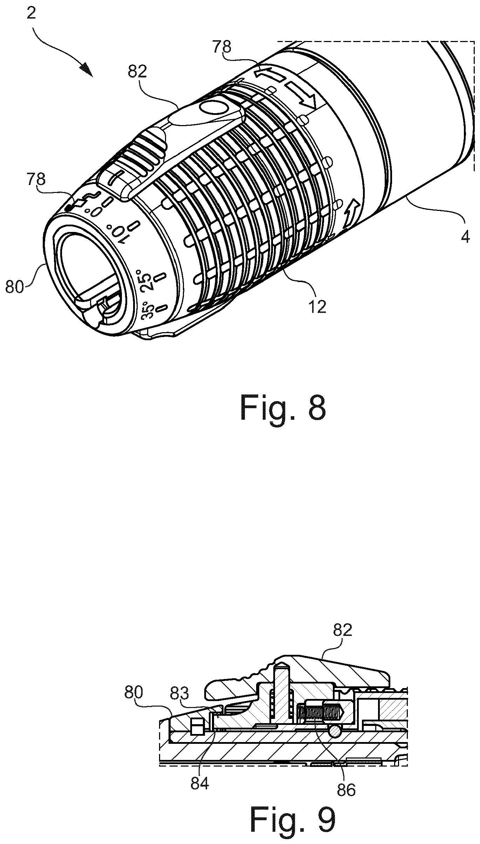

Fig. 8 zeigt eine Perspektivansicht des distalen Endabschnitts des Motorhandstücks 2 gemäß der bevorzugten Ausführungsform, ohne dass der Schaft 10 mit dem Handgriffabschnitt 4 gekoppelt ist. Wie vorstehend beschrieben, bewirkt eine Drehung des Bedienelements 12 in der ersten Drehrichtung A ein Abwinkeln des Werkzeugs 8 und eine Drehung des Bedienelements 12 in der zweiten Drehrichtung B löst die Kupplung zwischen dem Werkzeug 8 und dem Schaft 10, bzw. dem distalen Schaftabschnitt 22, indem der Wirkeingriff zwischen der ersten Kupplungsvorrichtung 28 und der zweiten Kupplungsvorrichtung 30 gelöst wird. Zur Erleichterung der Bedienung sind an dem Bedienelement 12 Indikatoren 78 in Form von Pfeilen, welche die Drehrichtungen angeben, angebracht. In Kombination mit Indikatoren 78 auf einer Indikatorhülse 80, welche distal des Bedienelements 12 drehfest an dem Handgriffabschnitt 4 angebracht ist, kann der Verwender bei der Bedienung des Handinstruments 1 schnell erkennen, welche Drehrichtung welcher Funktion entspricht. Hierfür sind, wie in Fig. 8 gezeigt, auf einer Außenumfangsfläche der Indikatorhülse 80 verschiedene Winkelpositionen, welche jeweils einer definierten Drehung des Bedienelements 12 in der ersten Drehrichtung A und damit einem definierten Abwinkeln des Werkzeugs 8 relativ zu dem Schaft 10 entsprechen, sowie ein Schloss-Symbol, bzw. ein Symbol eines geöffneten Schlosses, aufgebracht. Der Verwender kann somit den einzelnen Drehrichtungen A, B die jeweilige Funktion, nämlich das Abwinkeln oder das Entkuppeln, zuordnen. Das Motorhandstück 2 mitsamt dem Bedienelement 12 ermöglicht somit eine intuitive Bedienung und Integration der beiden Funktionen.

Wie in Fig. 8 gezeigt, ist an dem Motorhandstück 2 gemäß der bevorzugten Ausführungsform ein Verriegelungsschieber 82 angeordnet. Der Verriegelungsschieber 82 ist dabei in Radial- und in Umfangsrichtung fest so in dem Bedienelement 12 aufgenommen, dass sich der Verriegelungsschieber 82 lediglich in Axialrichtung relativ zu dem Bedienelement 12 zwischen einer (distalen) Verriegelungs- und einer (proximalen) Entriegelungsposition bewegen kann. In der Verriegelungsposition kann das Bedienelement 12, wie nachstehend näher erläutert, nicht relativ zu dem Handgriffabschnitt 4 gedreht werden.

Hierfür verrastet in der Verriegelungsposition ein distaler Zapfenabschnitt 83 des Verriegelungsschiebers 82, wie in Fig. 9 gezeigt, mit einem in der Indikatorhülse 80 aufgenommenen Rastring 84. Der Rastring 84 weist eine Mehrzahl an über den Umfang verteilten Ausnehmungen auf, in welche der Zapfenabschnitt 83 in der Verriegelungsposition vorragt, um so das Bedienelement 12 in Umfangsrichtung relativ zu dem Handgriffabschnitt 4 festzulegen bzw. festzuhalten. Die Ausnehmungen des Rastrings 84 entsprechen dabei vorzugsweise den auf der Außenumfangsfläche der Indikatorhülse 80 angebrachten Indikatoren 78. Somit kann der Verwender einfach ein Abwinkeln des Werkzeugs 8 bei einem definierten Winkel einstellen und das abgewinkelte Werkzeug 8 bei diesem Winkel fixieren.

Um das Bedienelement 12 für eine Verstellung des Winkels oder zum Lösen der Kupplung relativ zu dem Handgriffabschnitt 4 drehen zu können, muss der Verriegelungsschieber 82 folglich aus der Verriegelungsposition in die Entriegelungsposition verlagert werden, d.h. in Richtung proximal.

Bei dem Motorhandstück 2 gemäß der bevorzugten Ausführungsform ist der Verriegelungsschieber 82 in Axialrichtung über ein Federelement 86 gegen das Bedienelement 12 vorgespannt. Das Federelement 86 ist dabei so zwischen dem Verriegelungsschieber 82 und einer Anschlagsfläche des Bedienelements 12 angeordnet, dass es den Verriegelungsschieber 82 in die Verriegelungsposition, d.h. in Richtung distal, drückt. Das Federelement 86 übt folglich eine automatische Rückstellkraft auf den Verriegelungsschieber 82 aus, um diesen in der Verriegelungsposition zu halten.

Alternativ ist es selbstverständlich auch denkbar, dass das Motorhandstück 2 kein Federelement 86 aufweist. Bei einem solchen Motorhandstück gemäß einer Modifikation der bevorzugten Ausführungsform mit manueller Rückstellung muss der Verwender den Verriegelungsschieber 82 zur Entriegelung in Richtung proximal ziehen und zur Verriegelung aktiv in Richtung distal drücken.

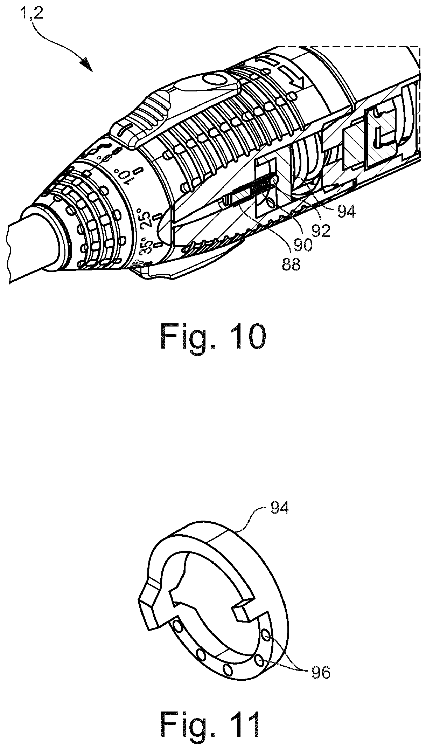

Wie in Fign. 10 und 11 gezeigt, ist bei dem Motorhandstück 2 gemäß der Modifikation der bevorzugten Ausführungsform ein zusätzliches Kugeldruckelement 88 vorgesehen. Dieses weist ein Federelement 90 auf, welches eine Kugel 92 in Richtung proximal gegen eine fest in dem Handgriffabschnitt 4 aufgenommene Abgleitkulisse (Kalottenrastring) 94 (siehe Fig. 11) drückt. Auf der Abgleitkulisse 94 sind kalottenförmige Rastausnehmungen 96 ausgebildet, welche die Kugel 92 zumindest teilweise aufnehmen können.

Die Rastausnehmungen 96 entsprechen, ähnlich wie die Ausnehmungen des Rastrings 84, definierten Winkeln, um welche das Werkzeug 8 relativ zu dem Schaft 10 bei einer bestimmten Verdrehung des Bedienelements 12 abgewinkelt werden kann. D.h. wenn das Bedienelement 12 um den definierten Winkel relativ zu dem Handgriffabschnitt 4 verdreht wird, dreht das Kugeldruckelement 88 mit dem Bedienelement 12 mit, bis der definierte Winkel erreicht ist, bei welchem die Kugel 92 aufgrund der Vorspannkraft des Federelements 90 in die entsprechende Rastausnehmung 96 der Abgleitkulisse 94 greift. Das zusätzliche Kugeldruckelement 88 ermöglicht folglich ein haptisches Feedback für den Verwender bei der Winkeleinstellung.

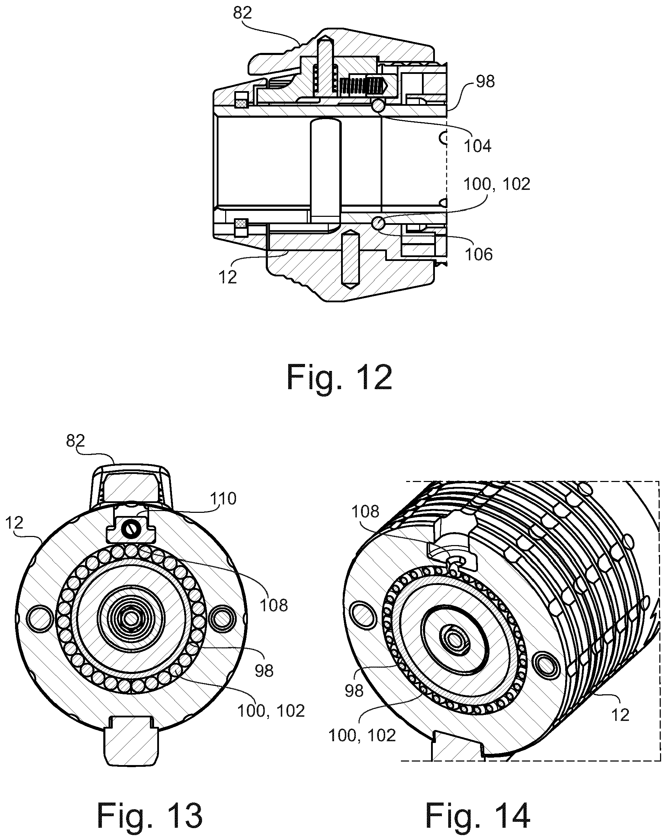

Fig. 12 zeigt eine Längsschnittansicht des Motorhandstücks 2 des medizinischen Handinstruments 1 gemäß der bevorzugten Ausführungsform. In Fig. 12 ist kein Schaft 10 in dem Motorhandstück 2 aufgenommen.

Wie in Fig. 12 zu erkennen, weist das Motorhandstück 2 bzw. der Handgriffabschnitt 4 an seinem distalen Endabschnitt ein Trägerelement 98 auf, welches in Form einer Hülse ausgestaltet ist und auf seiner radialen Innenseite bzw. in seinem Inneren einen Kupplungsabschnitt zur Aufnahme des Schafts 10 aufweist.

Auf einer radialen Außenseite des Trägerelements 98 ist das Bedienelement 12 angeordnet. Das Bedienelement 12 ist dabei, wie vorstehend erläutert, in Form einer Hülse ausgestaltet, deren Innendurchmesser im Wesentlichen größer ist als ein Außendurchmesser des Trägerelements 98. Mit anderen Worten ist das Bedienelement 12 so um das Trägerelement 98 angeordnet, dass sich zwischen dem Bedienelement 12 und dem Trägerelement 98 in radialer Richtung ein Spalt ausbildet. Das Bedienelement 12 kann demzufolge, wie vorstehend erwähnt, relativ zu dem Handgriffabschnitt 4, insbesondere relativ zu dem Trägerelement 98, gedreht werden.

Wie in Fig. 12 gezeigt, ist das Bedienelement 12 über zumindest eine Lagerstelle 100 drehbar auf dem Trägerelement 98 abgestützt. Die zumindest eine Lagerstelle 100 ist dabei offenbarungsgemäß als ein Wälzlager 102 ausgeführt. In der bevorzugten Ausführungsform ist das Wälzlager 102 ein Kugellager, bei welchem, wie vor allem in Fig. 13 zu erkennen, über den gesamten Umfang der Lagerstelle 100 Kugeln als Wälzkörper vorgesehen sind. D.h. die Lagerstelle 100 gemäß der bevorzugten Ausführungsform ist eine vollkugelige Lagerstelle.

Um einen besonders kompakten Aufbau der Lagerstelle 100 zu ermöglichen, ist eine Laufbahn eines Innenrings des Wälzlagers 102 in das Trägerelement 98 integriert. Hierfür ist auf einer Außenumfangsfläche des Trägerelements 98 eine umlaufende Nut 104 ausgebildet, in welcher die Wälzkörper des Wälzlagers 102 aufgenommen sind.

Analog dazu ist eine Laufbahn eines Außenrings des Wälzlagers 102 in das Bedienelement 12 integriert. D.h. auf einer Innenumfangsfläche des Bedienelements 12 ist eine umlaufende Nut 106 ausgebildet, in welcher die Wälzkörper des Wälzlagers 102 aufgenommen sind.

Fign. 13 und 14 zeigen Schnittansichten des Motorhandstücks 2 des Handinstruments 1 gemäß der bevorzugten Ausführungsform. Wie vorstehend erwähnt, ist die Lagerstelle 100 als vollkugelige Lagerstelle ausgeführt. D.h. über den gesamten Umfang der Lagerstelle 100 sind Wälzkörper angeordnet.

Wie in Fign. 13 und 14 zu erkennen, ist im Bereich des Verriegelungsschiebers 46 eine Einfüllöffnung 108 ausgebildet. Über diese Einfüllöffnung 108 können die Wälzkörper des Wälzlagers 102 eingefüllt werden. Anders ausgedrückt weist das Bedienelement 12 zur Montage der Wälzkörper die Einfüllöffnung 108 auf.

Nach der Montage der Wälzkörper wird die Einfüllöffnung 108, wie in Fig. 13 gezeigt, durch ein Verschlusselement 110 abgedeckt. Ein Herausfallen der Wälzkörper kann somit verhindert werden. Alternativ kann die Einfüllöffnung 108 auch durch den Verriegelungsschieber 82, insbesondere durch Komponenten eines Rückstellelements des Verriegelungsschiebers 82, abgedeckt werden, so dass hierfür keine zusätzlichen Komponenten vorgesehen werden müssen.

Vorstehend ist eine bevorzugte Ausführungsform des medizinischen Handinstruments 1 beschrieben. Dabei ist eine als Wälzlager 102 ausgebildete Lagerstelle 100 vorgesehen, über welche sich das Bedienelement 12 drehbar auf dem Trägerelement 98 des Handgriffabschnitts 4 abstützt. Alternativ kann sich das Bedienelement 12 jedoch auch über zwei Lagerstellen 100 auf dem Trägerelement 98 abstützen. Dies erlaubt es, eine mögliche Kippbewegung des Bedienelements 12 zu unterbinden und eine noch stabilere Lagerung mit geringerer Reibung zu realisieren.

Wie vorstehend erwähnt, wird als Wälzlager 102 bei dem medizinischen Handinstrument 1 gemäß der bevorzugten Ausführungsform ein Kugellager verwendet. Alternativ kann als Wälzlager 102 jedoch auch ein Nadel- oder Zylinderrollenlager verwendet werden. Solche Wälzlager ermöglichen es, die Kippbewegung auch mit einem einreihigen Lager effektiv zu unterbinden.

### Bezugszeichenliste

- 1: Handinstrument
- 2: Motorhandstück
- 4: Handgriffabschnitt
- 6: Antriebseinheit
- 8: Werkzeug
- 10: Schaft
- 12: Bedienelement
- 14: Verstellstift
- 16: Axialnut
- 18: Drehübertragungshülse
- 20: proximaler Schaftabschnitt
- 22: distaler Schaftabschnitt
- 24, 26: angestellte Stirnseite
- 28: erste Kupplungsvorrichtung
- 30: zweite Kupplungsvorrichtung
- 32: Werkzeugkopf / Effektor
- 34: Werkzeugschaft
- 36: Wälzlagereinheit
- 38: Antriebswelle
- 40: Wälzlager
- 42: Lagergehäuse
- 44: Axialsicherungsnut
- 46: Verriegelungskugel
- 48: Raststift
- 50: Außenrohr
- 52: Hohlrad
- 54: Innenverzahnung
- 56: Ritzel
- 58: Außenverzahnung
- 60: Sicherungsbuchse
- 62: Hohlwelle
- 63: Kante des proximalen Schaftabschnitts
- 64: Aufnahmebohrung
- 66: Wälzlager
- 68: Verstellbuchse
- 70: Silikonschlauch
- 72: Raststift-Aufnahmevertiefung
- 74: Vorspannelement
- 76: Rastvorsprung
- 78: Indikator
- 80: Indikatorhülse
- 82: Verriegelungsschieber
- 83: Zapfenabschnitt
- 84: Rastring
- 86: Federelement
- 88: Kugeldruckelement
- 90: Federelement
- 92: Kugel
- 94: Abgleitkulisse (Kalottenrastring)
- 96: Rastausnehmung
- 98: Trägerelement
- 100: Lagerstelle
- 102: Wälzlager
- 104: umlaufende Nut
- 106: umlaufende Nut
- 108: Einfüllöffnung
- 110: Verschlusselement

## Patentansprüche

1. Medizinisches Motorhandstück (2) zum Antreiben eines distalen Endeffektors (8), mit
einem Handgriffabschnitt (4),
einem vorzugsweise hülsenförmigen Bedienelement (12), das um eine Handgrifflängsachse drehbar an einem distalen Endabschnitt des Handgriffabschnitts (4) gehalten ist und mit dem Endeffektor (8, 22) derart koppelbar ist, um eine Drehbewegung des Bedienelements (12) in eine Bewegung des Endeffektors (8, 22) zu transformieren oder eine Funktion am Endeffektor (8, 22) zu bewirken,
**dadurch gekennzeichnet, dass**
das Bedienelement (12) über zumindest ein Wälzlager (102) drehbar an dem Handgriffabschnitt (4) abgestützt ist.

2. Medizinisches Motorhandstück (2) nach Anspruch 1, **dadurch gekennzeichnet, dass** das Motorhandstück (2) über einen Schaft (10, 20) mit dem Endeffektor (8, 22) koppelbar ist und das Bedienelement (12) bei einer Drehbewegung des Bedienelements (12) in einer ersten Drehrichtung die Bewegung des Endeffektors (8, 22), in Form eines Abwinkelns des Endeffektors (8, 22) relativ zu dem Schaft (10, 20), bewirkt.

3. Medizinisches Motorhandstück (2) nach Anspruch 2, **dadurch gekennzeichnet, dass** der Endeffektor (8, 22) eine erste Kupplungsvorrichtung (28) aufweist und der Schaft (10, 20) eine zweite Kupplungsvorrichtung (30) aufweist, welche in einem Kupplungszustand miteinander in Wirkeingriff stehen, wobei eine Drehbewegung des Bedienelements (12) in einer der ersten Drehrichtung entgegengesetzten, zweiten Drehrichtung den Wirkeingriff zwischen der ersten Kupplungsvorrichtung (28) und der zweiten Kupplungsvorrichtung (30) löst.

4. Medizinisches Motorhandstück (2) nach einem der vorhergehenden Ansprüche 1 bis 3, **dadurch gekennzeichnet, dass** das Bedienelement (12) drehbar auf einem, vorzugsweise als Hohlwelle ausgebildeten, Trägerelement (18) des Handgriffabschnitts (4) abgestützt ist.

5. Medizinisches Motorhandstück (2) nach einem der vorhergehenden Ansprüche 1 bis 4, **dadurch gekennzeichnet, dass** ein Außenring des Wälzlagers (102) einstückig mit dem Bedienelement (12) ausgebildet ist.

6. Medizinisches Motorhandstück (2) nach Anspruch 4 oder 5, **dadurch gekennzeichnet, dass** ein Innenring des Wälzlagers (102) einstückig mit dem Trägerelement (18) ausgebildet ist.

7. Medizinisches Motorhandstück (2) nach einem der vorhergehenden Ansprüche 1 bis 6, **dadurch gekennzeichnet, dass** das Bedienelement (12) eine Einfüllöffnung (108) zur Montage von Wälzkörpern des Wälzlagers (102) aufweist.

8. Medizinisches Motorhandstück (2) nach einem der vorhergehenden Ansprüche 1 bis 7, **dadurch gekennzeichnet, dass** das zumindest eine Wälzlager (102) als Kugel-, Nadel-, oder Zylinderrollenlager ausgeführt ist.

9. Medizinisches Motorhandstück (2) nach einem der vorhergehenden Ansprüche 1 bis 8, **dadurch gekennzeichnet, dass** das Bedienelement (12) über zwei Wälzlager (102) an dem Handgriffabschnitt (4) abgestützt ist.

10. Medizinisches Handinstrument (1) mit
einem medizinischen Motorhandstück (2) nach einem der vorhergehenden Ansprüche 1 bis 9 und
einem Endeffektor (8, 22), welcher über einen Schaft (10, 20) mit dem Motorhandstück (2) gekoppelt ist, um Drehmoment von dem Motorhandstück (2) auf den Endeffektor (8, 22) zu übertragen.
